# EUROPEAN PATENT APPLICATION

(11) **EP 1 815 835 A2**
(43) Date of publication of application: **08.08.2007**
(21) Application number: 07101684.4
(22) Date of filing: 02.02.2007
(51) Int. Cl.: A61G 11/00, A61F 7/00

(54) **Global emergency birthing bassinet**

(30) Priority: 06.02.2006 US 348059
(71) Applicant: General Electric Company, Schenectady, NY 12345 (US)
(72) Inventor: Ten Eyck, Lawrence Guy, Ellicott City, MD 21043 (US)
(74) Representative: Illingworth-Law, William Illingworth

(57) **Abstract**

A global, single use, emergency birthing bassinet (10) that can be transported to a location in an unassembled form and assembled on site. In the unassembled form, the bassinet is generally flat for easy transporting and has side and end portions (16, 18, 20, 22) that can be folded up to construct a three dimensional box-like structure for containing the infant. The bassinet (10) includes heating and cooling packs (32) that can be activated by the user to provide either heating or cooling to the infant. The bassinet (10) includes instructions (42) on the assembly and use of the various components in the appropriate language or easy to interpret pictograms that illustrate the instructions. In an embodiment for disadvantaged areas, the bassinet includes a clean birthing kit (44) containing various components to maintain a clean environment during the birthing of the infant. In an embodiment for more advanced locations there can also be temperature (34) and timing devices (38).

## Description

### BACKGROUND

The present invention relates to a bassinet for the birthing of an infant, and, more particularly, to an emergency bassinet that can be used in remote locations to provide suitable conditions for the birthing of a child.

In middle income and low income countries, the majority of births occur in the home setting. These births are usually attended by a traditional birth assistant (TBA), normally another woman, and, sometimes, a mid-wife. Rarely is the person attending the birth a skilled physician.

The mother or TBA relies on locally acquired knowledge or previous birthing experience to care for the newborn. In low income countries, access to skilled healthcare workers is severely limited. Births mostly happen in the home in sub-optimal and even dangerous conditions where the lack of water and heat prevents access to sterilized equipment to manage the birth. Unwashed hands and a lack of sterile instruments to cut the umbilical cord and sterile string to tie off the cord lead to post birth infections that can jeopardize the infant and mothers survival. This setting has the highest rates of neonatal mortality in the world.

Worldwide each year, an estimated 4 million newborns die in the first four weeks of life. A similar number are stillborn. Approximately ¾ of the deaths in the first 4 weeks happen within the first week of life. Globally the main direct causes of neonatal death are estimated to be: pre-term birth (28%); severe infections (26%) and asphyxia (23%).

In this environment, thermal management is critical and keeping the infant warm can reduce infant mortality. Resuscitation for asphyxia or "no cry at birth" due to prematurity is also manageable with minimal training. Many researchers feel that simple resuscitation efforts can reduce the number of asphyxia related deaths. The simple methods include airway clearance and thermal management which may mean cooling the body for a period of time in order to protect the brain of the newborn.

Even in high level care settings, neonatal asphyxia remains a significant contributor to neonatal mortality and morbidity. Recently, brain and/or total body cooling has been shown to decrease the cascade of biologic events leading to brain cell death. It is important to start cooling as soon as feasible. The window of time is probably limited to a few hours. This has, heretofore, meant transfer of the asphyxiated neonates to a Neonatal Intensive Care Unit (NICU) where the head cooling or total body cooling devices are located but at the cost of taking time to make the transfer and which passage of time could further exacerbate the problem of the infant.

Accordingly, it would be advantageous to have an infant emergency bassinet that could be used where the facilities to properly carry out the birthing of an infant are simply not adequate and also to have a bassinet that can facilitate the birthing of an infant in more advanced settings. It would be further advantageous to have an infant bassinet that includes various supplies that can be provided with the bassinet and which can be used in carrying out the birthing of the infant under improved cleanliness conditions.

### SUMMARY OF THE INVENTION

Accordingly, with the present invention, there is a global, single use emergency bassinet that is initially supplied in an unassembled, generally flattened form so that it can be easily shipped to locations around the globe. As such, one of the advantageous features of the present invention is that it is constructed of reasonably inexpensive materials and, in its unassembled form, can be shipped to remote locations such as disadvantaged or low income countries for use there without a large expenditure of funds so as to be available in those areas of the globe where sorely needed to improve the safety of both the newborn infant as well as the birthing mother.

Therefore, large quantities of the present emergency bassinets can be shipped to locations where the bassinets are needed to improve conditions surrounding the birth of an infant. Once at or near the location of use, the present emergency basinet can be readily converted to an assembled form of a three-dimensional box-like structure for use with a newly born infant.

In the exemplary embodiment, the unassembled form can include side and end portions that extend outwardly from the main infant support portion and there can be a hinge area, such as a living hinge intermediate the main infant support portion and the side and end portions. As such, the user can simply follow instructions printed or enclosed in each emergency bassinet, in the proper language or languages for the area, or in easy to interpret pictograms, to fold the side and end portions along the hinge areas to construct a three-dimensional box-like structure thereby forming a clean compartment for locating the newborn infant.

Once assembled, there are carrying handles that enable the transportation of the infant from one location to another. The emergency bassinet also contains biodegradable chemical heating and cooling packs to rapidly warm or cool a newborn infant located within the assembled compartment of the bassinet.

In the global health embodiment of the present invention, there is included a clean birthing kit to improve the sanitary conditions of the birth. The clean birthing kit includes a number of devices and components assembled together to assist in the birthing of the infant in the proper surroundings. The clean birthing kit comprises various components, however, as a minimum, includes surgical sheets, a sterile string to tie off the umbilical cord, a razor for cutting the umbilical cord, disposable, biodegradable gloves, a biodegradable biohazard bag into which the user can place the placenta as well as blood contaminated sheets and instruments, a disposable fetal scope, nasal bulb and DeLee suction kit for aspirating fluids from the airway, a mask for mask-to-mouth resuscitation, aseptic hand washing wipes, a candle and waterproof matches to provide light, chemical tablets to purify water and a polyester blanket for wrapping the infant.

In the exemplary embodiment intended for a more developed market or high level care settings, the clean birthing kit may not be necessary, however, on the other hand, there can be certain more sophisticated components contained or included within the present emergency bassinet, once assembled, such as, for example, a digital timer, and a temperature monitor for monitoring the cooling and warming times and the skin temperature of the neonate. As such, even in the more advanced facilities, the user can immediately start the heating or cooling process without the need to move the infant to that NICU unit, thereby saving valuable time. In addition, because resuscitation warmers and traditional bassinets are readily available in the developed world, the present invention designed is intended to be nested into the surface of traditional resuscitation tables. This allows the infant to be immediately cooled while other care procedures are being conducted.

In essence, the present emergency bassinet provides a novel approach to solving one of the world's most pressing child health issues. It presents developing health systems with a low cost rudimentary resuscitation capability and components to provide a clean birthing environment for use by a TBA or lower skilled healthcare workers. The technologies employed do not require electricity and, as such, are suitable for global markets where access to water, heat and electricity are not available.

These and other features and advantages of the present invention will become more readily apparent during the following detailed description of embodiments, provided by way of example, taken in conjunction with the drawings herein.

### BRIEF DESCRIPTION OF THE DRAWINGS

Figure 1 is a schematic view of the emergency bassinet of the present invention its unassembled, generally flattened form; and
Figure 2 is a schematic view of the emergency bassinet in its assembled form and illustrating the various devices that are included therewith.

### DETAILED DESCRIPTION OF THE INVENTION

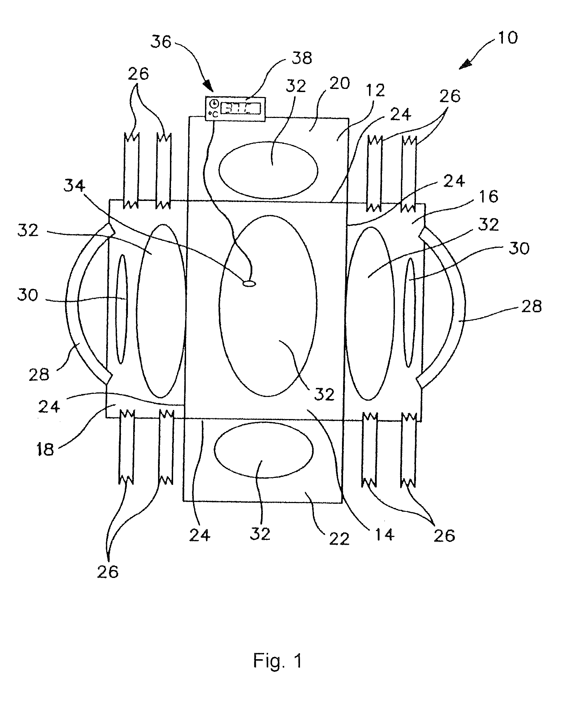

Referring now to Figure 1, there is shown a schematic view of the emergency bassinet 10 in its unassembled, generally flattened form. As can be seen the enclosure 12 comprises a central infant support portion 14 that is adapted to underlie an infant when the ultimate bassinet 10 is in use. The material used for the enclosure 12 is preferable a hard cardboard or other inexpensive material to keep the cost of the emergency bassinet 10 as low as possible and yet be strong enough to safely provide support and transport for a newborn. Other materials can be used such as other fiber or cellulose based materials or plastic materials, however, it is preferable for the material to be sustainable and yet biodegradable. When in the unassembled form, the emergency bassinet 10 is configured to be easily shipped in large quantities to the ultimate destination and to be easily stored.

Extending outwardly from the central infant support portion 14 are side portions 16, 18 and end portions 20, 22. Preferable the side portions and end portions are joined to the central infant support portion 14 by means of a weakened line or score line, (including a living hinge), forming hinges 24. As will be seen, therefore, the hinges 24 facilitate the assembly of the emergency bassinet 10 by providing a line along which the side portions 16, 18 and end portions 20, 22 can be folded. The use of multiple score lines will enable the user to assemble the emergency bassinet 10 so as to be the proper size for the particular infant. As show in Fig., 1, the side portions 16, 18 and the end portions 20, 22 extend outwardly from the central infant support portion 14, however, the side portions 16, 18 and end portions 20, 22 may also be folded inwardly in the unassembled form such that they overlap the central infant support portion 14.

Extending from each of the side portions 16, 18 are straps 26 that have hook and loop fastening system (Velcro) used thereon, that is, the straps 26 can have the hooks formed thereon with corresponding loop straps affixed to the underside of the end portions 20, 22 (not shown). As can be appreciated, while the straps 26 are shown affixed to the side portions 16, 18, they can just as easily be affixed to the end portions 20, 22 or other types of securing means can be used to join together the adjacent edges of the end portions 16, 18 to the end portions 20, 22 in a manner and for a purpose that will be later explained.

As also can be seen, there are carrying handles to enable the user to easily carry the emergency bassinet 10 from location to location and may comprise carrying straps 28 and/or handle cutouts 30. The carrying straps 28 can be nylon for strength and lightness, however more preferably, the carrying straps 28 are of a material that is sustainable and yet biodegradable. The cutouts 30 can be used by two people to transport the infant within the emergency bassinet 10 while the carrying straps 28 allow the transportation of the infant by only one person.

There are also chemical heating or cooling packs 32 provided and affixed to the upper surfaces (as shown in Fig. 1) of the central infant support portion 14 and the side portions 16, 18 and end portions 20. Again there may be a lesser or great number of the chemical heating or cooling packs 32 that are provided with the emergency bassinet 10 and the actual orientations and locations may vary.

In general, the chemical heating or cooling packs 32 contain biodegradable, human and environmentally safe chemicals which, when mixed with initiating chemicals, will become cold (33 degrees C) or warm (36.5 degrees C). The chemical heating or cooling packs 32 can preferably contain both warm and cold initiating chemical reaction starters such that a user can break the appropriate capsule to initiate the desired reaction. The chemical reaction can hold the temperature for about two hours and, once the heat or cooling pack 32 has been spent, the heating or cooling pack 32 can remain soft and pliable so as to be used as a mattress for the infant. There are, of course, instructions on the heating or cooling packs 32 that inform the user as to how to initiate the desired reaction in the language of the intended use of the emergency bassinet 10, in a variety of languages, or in the form of readily understood pictograms that illustrate to the user the activation and use of the heating or cooling packs 32.

As will be understood, the present emergency bassinet 10 is usable in remote, middle and low income countries but also in more sophisticated environments and, therefore, there are certain components that may be included in the embodiment intended for the more advanced infant care facilities. To that end, there is shown in Fig. 1, certain components that may be present in the emergency bassinet 10 utilized in the more advance facilities. Those components may include a temperature probe 34 that is adapted to be affixed to the skin of the infant to sense the skin temperature of that infant. The signal from the temperature probe 34 indicative of the infant's skin temperature is communicated to a digital thermometer 36 to display that temperature to a user of the emergency bassinet 10.

Along with the digital thermometer 36, there may be a timer 38 provided so that the user can monitor or ascertain the times of the various heating or cooling cycles administered to the infant. There may of course be other components, equipment or devices included with the embodiment of the present invention intended for the more advanced infant birthing locations.

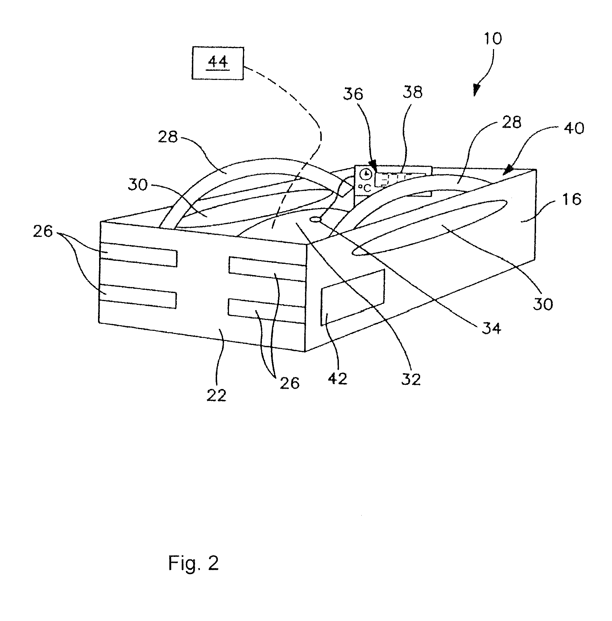

Turning now to Fig. 2, there is shown a schematic view of the emergency bassinet 10 in its assembled form. As can be seen, the assembly for the emergency bassinet 10 has been accomplished by folding the side portions 16, 18 and the end portions 20, 22 upwardly to form a three dimensional box-like structure that has an internal infant compartment 40 for contain the newborn infant in clean surroundings. The side portions 16, 18 and end portions 20, 22 are secured in the assembled form by use of the straps 26 having Velcro surfaces. There is also indicia 42 printed on one of the external surfaces of the assembled emergency bassinet 10 to inform a user, either in the appropriate language for the intended destination of in a number of languages, as to the assembly of the emergency bassinet 10 as well as to its use and the use of the components and material contained therein. The indicia 42 can be in the form of pictograms for persons of all educational levels to understand the use of the bassinet without the need to have instructions in any particular language.

Contained within or accompanying the embodiment intended for middle/low income countries is a clean birthing kit 44 that can be used in the delivery of the infant in order to maintain clean and proper surroundings for that birth. According, the clean birthing kit 44 contains various components and devices that improve the birthing process in order to improve the survival rate of the infant as well as the mother.

As a minimum, the clean birthing kit 44 will include surgical sheets, a sterile string to tie off the umbilical cord, a razor for cutting the umbilical cord, disposable, biodegradable gloves, a biodegradable biohazard bag into which the user can place the placenta, as well as blood contaminated sheets and instruments, a disposable fetal scope, nasal bulb and DeLee suction kit for aspirating fluids from the airway, a mask for mask-to-mouth resuscitation, ascetic hand washing wipes, a candle and waterproof matches to provide light, chemical tablets to purify water and a polyester blanket for wrapping the infant. Preferable as many of the above items, that is, to the extent possible, are biodegradable.

In an exemplary embodiment, the clean birthing kit is a flat package enclosed with the emergency bassinet 10 and a part thereof and the clean birthing kit 44 is packaged in a way that allow the components to be used in the proper birthing order, i.e. clean birthing sheets on top. There can be step-by step pictogram instruction printed on the external surface of the clean birthing kit 44, or the enclosure 12 to guide the user in the use of each of the devices contained therein.

Thus for such countries, the emergency bassinet 10 not only provides clean surroundings for the newborn with heating or cooling that can be used to treat the infant but also contains certain components and devices to assist in the birthing process and to improve the cleanliness of the environment of that birth, thereby contributing to the safety of the mother and child.

Those skilled in the art will readily recognize numerous adaptations and modifications which can be made to the emergency bassinet of the present invention which will result in an improved emergency bassinet, yet all of which will fall within the scope and spirit of the present invention as defined in the following claims. Accordingly, the invention is to be limited only by the following claims and their equivalents.

## Claims

1. A bassinet (10) for facilitating the birthing of an infant comprised of an enclosure (12) to contain an infant after birth, the enclosure (12) having an unassembled, generally flattened form and an assembled form forming a three dimensional box-like structure having carrying straps (28), the assembled form having an infant support section (14) providing a clean area for resting an infant and at least one of a cooling pack (32) and a heating pack (32) adapted to be activated by a user.

2. The bassinet (10) as defined in claim 1 wherein the enclosure includes side portions (16, 18) and end portions (20, 22) that are foldable to form the three dimensional box-like structure.

3. The bassinet (10) as defined in claim 2 wherein the side portions (16, 18) and end portions (20, 22) include straps (26) having hook and loop fastening devices to retain the side portions (16, 18) and end portions (20, 22) in the assembled form.

4. The bassinet (10) as defined in claim 1 wherein the enclosure (12) is comprised of a biodegradable structural cardboard or cellulose material.

5. The bassinet (10) as defined in claim 1 further including instructions (42) printed on the enclosure in an appropriate language or in the form of pictograms to assist a user in assembling the bassinet (10).

6. The bassinet (10) as defined in claim 1 further including a clean birthing kit (44) that includes at least one of the following components: surgical sheets, a sterile string to tie off the umbilical cord, an instrument for cutting the umbilical cord, disposable gloves, a biohazard bag, a disposable fetal scope, a nasal bulb and suction kit for aspirating fluids from the airway, a resuscitator, hand washing wipes, a light source, chemical tablets to purify water and a blanket for wrapping the infant.

7. A bassinet (10) for facilitating the birthing of an infant comprised of an enclosure (12) to contain an infant after birth, the enclosure (12) having an unassembled, generally flattened form and an assembled form forming a three dimensional box-like structure, the assembled form having an infant support section (14) providing a clean area for resting an infant, a cooling/heating pack (32) activatable to cool or heat an infant and at least one of a temperature probe (34) with a digital thermometer (36) and a timer (38).

8. The bassinet (10) of claim 7 wherein the bassinet includes a temperature probe (34) adapted to be affixed to the skin of an infant, a digital thermometer (36) to display the skin temperature of the infant sensed by the temperature probe and a timer (38).

9. A method of providing a bassinet (10) for birthing of an infant comprising the steps of:
providing an enclosure (12) in a flattened, unassembled form having components to assist in the birthing of an infant including a heating/cooling pack (32),
transporting the bassinet (10) to a user site in an unassembled form ; and
assembling the bassinet (10) at or proximate to the intended site of use.

10. The method as defined in claim 9 wherein the step providing a bassinet (10) comprises providing an enclosure (12) comprised of a single use, inexpensive material having a central infant support section (14) and side portions (16, 18) and end portions (20, 22) extending therefrom and wherein the step of assembling the bassinet (10) comprises folding the side portions (16, 18) and end portions (20, 22) upwardly to form a three dimension box-like structure with the central infant support section (14) adapted to support the infant in a clean environment.
